# EUROPÄISCHE PATENTSCHRIFT

(11) **EP 0 663 213 B1**
(45) Veröffentlichungstag und Bekanntmachung des Hinweises auf die Patenterteilung: **21.03.2001**
(21) Anmeldenummer: 94100657.9
(22) Anmeldetag: 18.01.1994
(51) Int. Cl.: A61L 2/06, A61L 2/26

(54) **Sterilisationskammer zur Dampfsterilisation von ärztlichen Instrumenten, Implantaten und ähnlichem**
Sterilization chamber for steam sterilization of surgical instruments, implants and the like
Enceinte de stérilisation pour la stérilisation à vapeur d'instruments chirurgicaux, implants et similaires

(43) Veröffentlichungstag der Anmeldung: 19.07.1995
(73) Patentinhaber: SCI-CAN, A DIVISION OF LUX AND ZWINGENBERGER LTD., Toronto, Ontario M2J 1RJ (CA)
(72) Erfinder: Zwingenberger, Arthur, Toronto, Ontario M4W 2R6 (CA); Saupe, Martin, D-63075 Offenbach (Main) (DE)
(74) Vertreter: Puschmann, Heinz H., Dipl.-Ing.(FH)

(56) Entgegenhaltungen:
- FR-A- 2 597 185
- US-A- 4 824 644

## Beschreibung

Die Erfindung betrifft ein Druckgehäuse für eine Sterilisationskammer für die Aufnahme eines Sterilisationsgefäßes.

Die Kostendämpfung in der Medizin und die Vermeidung von Sondermüll erfordert die Abkehr von sogenannten Einweg-Instrumenten und damit die Wiederzuwendung zu deren Mehrfachgebrauch, was die Wiedereinführung der Sterilisationstechnik bedingt. Hierzu sind an die Stelle aufwendiger Autoklaven energiesparende Kleingeräte getreten, mit deren Hilfe die jeweils benötigten Instrumente am Ort ihrer Anwendung schnell und kostengünstig zu sterilisieren sind; vgl. EP 0429 960.

Bei der bekannten Anordnung ist das Sterilisationsgefäß als lose Kassette ausgebildet, deren Boden- und Deckelteil über flanschartige Ränder und Dazwischenfügung einer Dichtung während des Sterilisationsvorganges in einer Haltevorrichtung dicht zusammengepreßt gehalten sind.

Die Ausbildung des Sterilisationsgefäßes als zweiteilige, allseitig gegenüber der Atmosphäre geschlossene Kassette hat sich jedoch in der Herstellung als aufwendig erwiesen. Ferner sind enge Toleranzen bei der Herstellung einzuhalten, da beim Einschieben der Kassette in die Haltevorrichtung ein Kuppeln mit den zugehörigen Zu- und Ableitungen erforderlich ist.

Es besteht daher Bedarf einer weiteren Vereinfachung solcher Geräte, insbesondere hinsichtlich des Aufbaus des notwendigen Druckgehäuses zur Aufnahme des Sterilisationsgefäßes und dessen Beschickung mit zu sterilisierenden Instrumenten bei gleichzeitiger Senkung der Gestehungskosten.

Einer wohlfeilen Ausbildung der Druckgehäuse solcher Sterilisationskammern stehen aber die relativ engen Toleranzen für den funktionell unabdingbaren druckdichten Abschluß des Sterilisationsraumes entgegen, der trotz thermisch stark wechselnder Belastungen auch nach längerem Gebrauch gewährleistet sein muß, anderenfalls die erforderliche Keimfreiheit der zu sterilisierenden Instrumente nicht gewährleistet ist.

Aufgabe der Erfindung ist es daher, ein technisch einfach ausgebildetes, den medizinischen Anforderungen genügendes Druckgehäuse zu schaffen, dessen Ausbildung eine rationelle und maßgenaue Fertigung ermöglicht, das über lange Betriebszeiten einsatzfähig ist und eine weitere Vereinfachung der Bedienung des Sterilisationsgerätes ermöglicht.

Diese Aufgabe ist gemäß der Erfindung dadurch gelöst, daß die Sterilisationskammer Ober-, Unter- und Seitenwandungen aus einzelnen losen, wärmeisolierenden Platten aufweist, die in zentrischen Durchbrüchen mindestens zweier identisch ausgebildeter, in einem Träger formschlüssig gehaltener Rahmenteile angeordnet und an den Innenkanten der Durchbrüche anliegend formschlüssig gehalten sind und daß die als Oberwandung dienende Platte des Druckgehäuses eine Metallfassung trägt zwecks Aufnahme einer federnd in ihrer unwirksamen Lage gehaltenen Abdeckung mit einer eine öffnung für den Dampfübertritt aufweisenden Scheibe als Träger einer ihren Randbereich bildenden, mit dem freien oberen Rand des von der Sterilisationskammer umfaßten Sterilisationsgefäßes korrespondierenden elastischen Gummidichtung.

Nach einem weiteren Merkmal der Erfindung tragen die die Rahmenteile der einen Außenkante zugeordnete voneinander abgewandte hakenförmige Fortsätze, denen in ihren Längenausdehnungen gestufte Durchbrüche des Trägers derart zugeordnet sind, daß die Fortsätze nach dem Einsetzen in den jeweils längeren Teil der Durchbrüche und nach Verschieben quer zu den Durchbrüchen (y-Richtung) jeweils den kürzeren Teil der Durchbrüche untergreifen und in dieser Lage durch Fortsätze an der Unterwandung der Teil der Sterilisationskammer bildenden losen Platte, die in entsprechende Durchbrüche in dem Träger eingreifen, gehalten sind.

Hierbei entsprechen die maximalen Breiten der Durchbrüche der doppelten Materialstärke der Rahmenteile und die Länge der längeren Teile der Breite der hakenförmige Fortsätze und die Länge der kürzeren Teile der Durchbrüche der Breite der Basis der Fortsätze.

Zwecks sicherer Halterung der Wandungen an den Trägern weisen nach einem weiteren Merkmal der Erfindung die Platten die Innenkanten der Durchbrüche der Rahmenteile im montierten Zustand umgreifende Nuten auf.

Weitere Merkmale der Erfindung ergeben sich aus den Unteransprüchen.

Der Erfindung liegt der verblüffend einfache Gedanke zu Grunde, für das Druckgehäuse als Teil der Sterilisationskammer lediglich Seiten-, Ober- und Unterwandungen vorzusehen, diese Wandungen als einzelne Platten auszubilden und diese an ihren Randbereichen gegeneinander verspannt in den Durchbrüchen von Rahmenblechen anzuordnen. Die zur Erzielung der für den Sterilisationsprozeß erforderlichen Druckdichtigkeit des einerseits vom Sterilisationsgefäß und andererseits von der Sterilisationskammer gebildeten Sterilisationsraumes notwendigen Toleranzen werden hierbei erfindungsgemäß durch einfachst zu fertigende und zu montierende ebene Einzelteile sichergestellt.

Darüber hinaus wird durch das erfindungsgemäße Druckgehäuse die Betriebssicherheit von Dampfsterilisationsgeräten wesentlich gesteigert, da bei Zuführung von Dampf in das Druckgehäuse die Abdeckung entgegen der Wirkung der diese in der Offenstellung haltenden Federn druckdicht gegen die Oberkanten des Sterilisationsgefäßes gedrückt und dort gehalten wird bis die Dampfzufuhr unterbrochen ist. Ein weiterer Vorteil der erfindungsgemäßen Ausbildung liegt darin, daß bei Unterbrechung der Dampfzufuhr zum Zwecke der Entnahme der sterilisierten Instrumente das Sterilisationsgefäß selbsttätig freigegeben wird. Das Sterilisationsgefäß kann also im geöffneten Zustand der Sterilisationskammer entnommen werden, was für die Entnahme und Beschickung mit ärztlichen Instrumenten, Implantaten und ähnlichem einen erheblichen Bedienungskomfort mit sich bringt. Auch kann der Wechsel eventuell beschädigter Wandungen vom Bedienungspersonal selbst - also ohne die Inanspruchnahme von speziell ausgebildetem Servicepersonal - rasch durchgeführt werden.

Die erfindungsgemäße Ausbildung bringt eine erhebliche Gewichtsersparnis mit sich. Ein weiterer Vorteil der Erfindung liegt darin, daß durch die Wahl geeigneter Materialien, vorzugsweise thermisch isolierende Kunststoffe für die Wandungen der Sterilisationskammer und Metall für die Rahmenteile auf eine weitere Isolierung des Sterilisationsraumes gegenüber den diesen umgebenden Bauteilen verzichtet werden kann. Insbesondere ist für die Materialwahl der Wandungen zwingend zu berücksichtigen, daß keine Gesundheitsgefährdung durch Kontaminierung des zu sterilisierenden Gutes durch Materialbestandteile - wie etwa Mineralfasern - auch nach längerem Gebrauch entsteht.

Bei der Wahl des Materials der Wandungen der Sterilisationskammer ist selbstverständlich darauf zu achten, daß das Material eine möglichst geringe Neigung zur Wasseraufnahme, hohe Temperaturbeständigkeit und Formstabilität aufweist.

Ferner ist für das Wandungsmaterial nach Möglichkeit wieder in den Materialkreislauf einleitbares, also recyclebares Material vorzusehen.

Die Erfindung ist nachfolgend anhand eines in der Zeichnung mehr oder minder schematisch dargestellten Ausführungsbeispiels beschrieben.

Es zeigen:
- Fig. 1: das Gesamtschema einer bekannten Anordnung zum Sterilisieren von ärztlichen Instrumenten und Implantaten mit einem in einer Haltevorrichtung angeordneten Sterilisationsgefäß,
- Fig. 2: eine perspektivische Darstellung eines gemäß der Erfindung ausgebildeten Sterilisationsgefäßes zur Aufnahme von ärztlichen Instrumenten und Implantaten für die Einbringung in ein den Gegenstand der Erfindung bildendes, nur teilweise dargestellten Druckgehäuses einer Sterilisationskammer,
- Figur 3: eine teilweise im Schnitt dargestellte Vorderansicht des ein Teil der Sterilisationskammer bildenden erfindungsgemäßen Druckgehäuses,
- Figur 4: eine perspektivische Darstellung der Wandungen der das Druckgehäuse nach Figur 2 bildenden Platten,
- Figur 5: eine perspektivische Ansicht des die Druckgehäuse nach Figur 2 haltenden Trägers und
- Figur 6: eine Einzelheit aus Figur 5 in vergrößertem Maßstab.

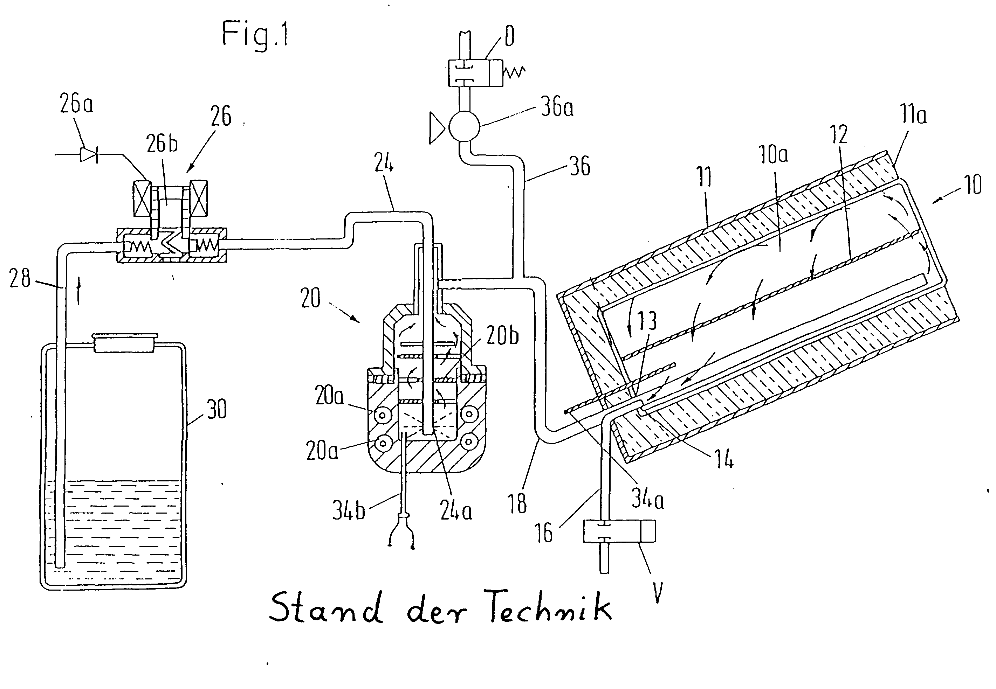

Wie Figur 1 zeigt, ist zur Dampf-Sterilisation von ärztlichen Instrumenten und Implantaten ein zur Waagerechten geneigtes Sterilisationsgefäß 10 vorgesehen, das in einer Ausnehmung einer ortsfest angeordneten Haltevorrichtung 11 eingeschoben werden kann, die eine Wärmeisolierung 11a aufweist. Zur Aufnahme der zu sterilisierenden ärztlichen Instrumente und Implantate weist das Sterilisationsgefäß eine als Lochplatte ausgebildete Aufnahmeplatte 12 auf. Die Haltevorrichtung 11 weist eine durch eine Auslaßöffnung 13 im Sterilisationsgefäß ragende Luft- und Kondensat-Auslaßleitung 16 und eine durch eine Einlaßöffnung 14 in das Sterilisationsgefäß ragende Dampfzuleitung 18 auf, so daß diese im Abstand voneinander in den von dem Sterilisationsgefäß umschlossenen Raum 10a ragen.

Die Luft- und Kondensat-Auslaßleitung 16 ist über ein Ventil V mit Atmosphäre verbindbar, während die Dampfzuleitung 18 mit einem Dampfgenerator 20 verbunden ist. Die Dampfkammer des Dampfgenerators 20 wird über elektrische Heizelemente 20a aufgeheizt und über eine Speiseleitung 24, die an ihrem Ende Austrittsöffnungen 24a aufweist, pulsartig mit Wasser versorgt, wobei über im Innern der Dampfkammer angeordnete Prallplatten 20b ein direktes Einspritzen von siedendem Wasser in die Dampfzuleitung 18 verhindert wird.

In die den Dampfgenerator mit Wasser versorgende Speiseleitung 24 ist eine Dosierpumpe 26 eingefügt, die über eine Saugleitung 28 mit einem Behälter 30 kommuniziert, der destilliertes Wasser enthält. Der Antrieb der Dosierpumpe 26 erfolgt über einen von einem über eine Diode 26a gespeisten Elektromagneten betätigten Kolben 26b. Über Thermofühler 34a und 34b werden die Temperaturen in der Dampfkammer des Dampfgenerators und in dem Sterilisationsgefäß gemessen.

Schließlich kann die Sterilisationskammer über eine Abzweigleitung 36 und einen Druckregler 36a mit einer nicht dargestellten, über ein Ventil D anschaltbaren Druckluftquelle verbunden werden, um am Ende eines Sterilisationsvorganges die Instrumente und Implantate zu kühlen und zu trocknen.

Über eine nicht dargestellte Steuereinrichtung wird der Ablauf eines Sterilisationsvorganges - bei dem der aus der Dampfzuleitung austretende Dampfstoß schwallartig das Sterilisationsgefäß durchläuft - gesteuert. Diese und das hierzu gehörende Verfahren für den Betrieb der beschriebenen Anordnung bilden nicht den Gegenstand der Erfindung und sind in der eingangs genannten EP 0 429 960 A2 dargestellt und beschrieben.

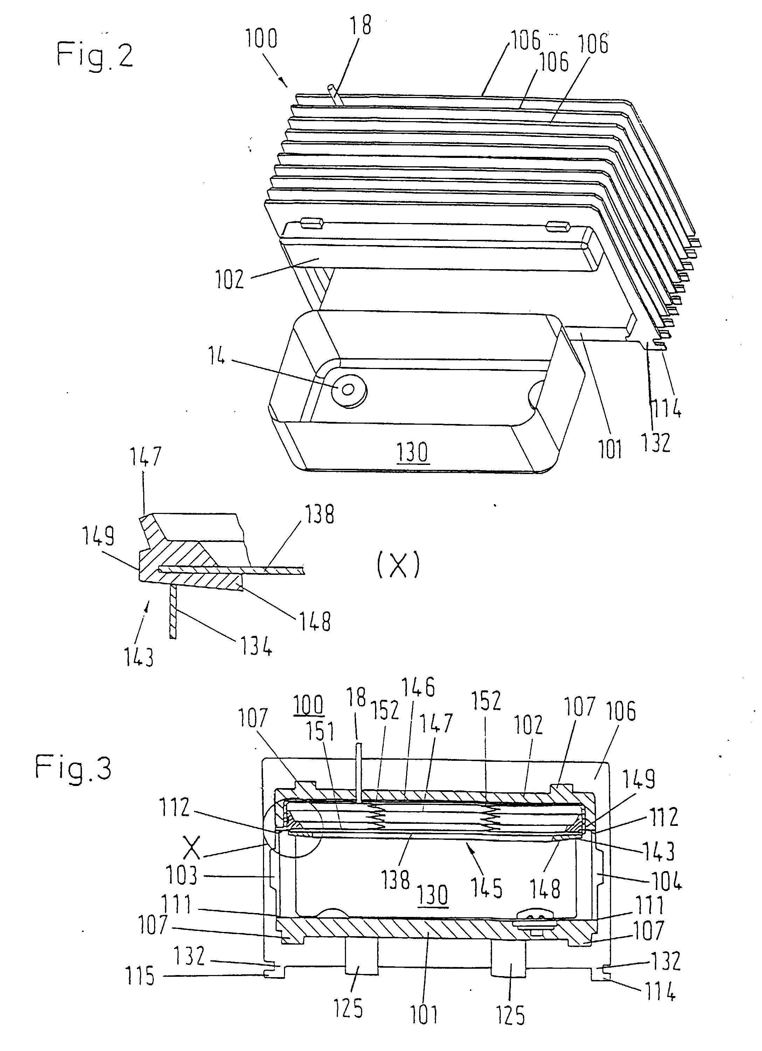

Nunmehr sei das an die Stelle der Haltevorrichtung 11 tretende, insgesamt mit der Bezugsziffer 100 bezeichnete, Teil einer Sterilisationskammer bildende Druckgehäuse in Verbindung mit den Figuren 2 bis 6 beschrieben.

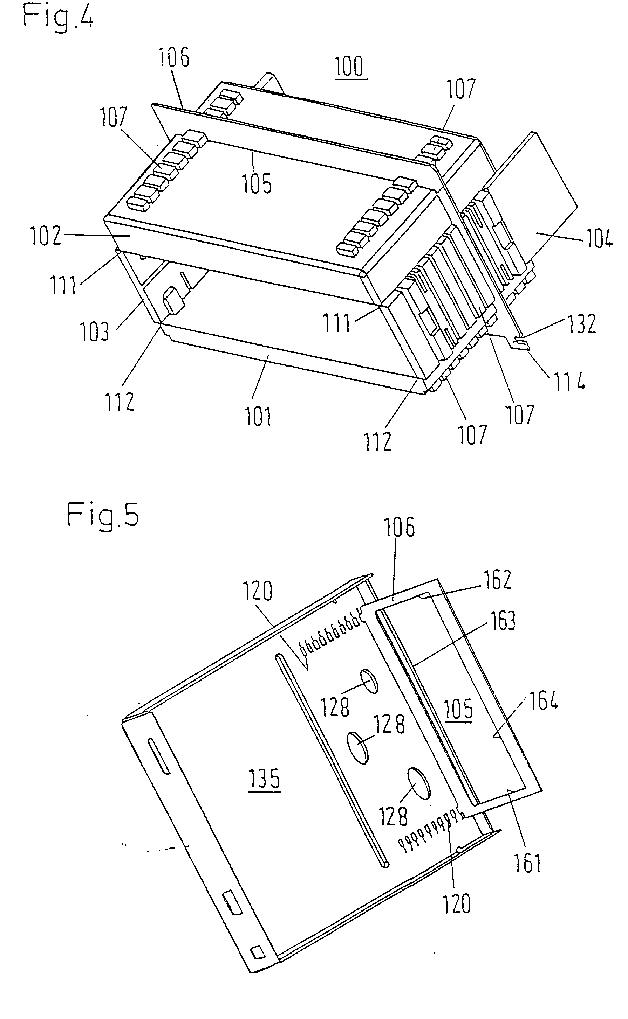

Das Druckgehäuse 100 umfaßt, wie insbesondere Figur 4 zeigt, vier als lose Platten 101, 102, 103 und 104 ausgebildete Wandungen, welche in Anlage an den Innenkanten von zentrischen Durchbrüchen 105 von Rahmenteilen 106 gehalten sind, wie dies Figur 3 besonders deutlich zeigt. Hierzu dienen von an den nach außen weisenden Flächen der Platten angeordneten Vorsprüngen 107 gebildete Nuten, in die die Rahmenteile 106 eingreifen; vgl. Figur 4. Die die Wandungen des Druckgehäuses bildenden Platten 101 bis 104 sind derart in die Rahmenteile 106 eingebettet, daß die zusammenstoßenden Randflächen 111/112 der Platten 101/102 und 103/104 miteinander abschließen, wobei die Platten 103 und 104 Distanzplatten sind.

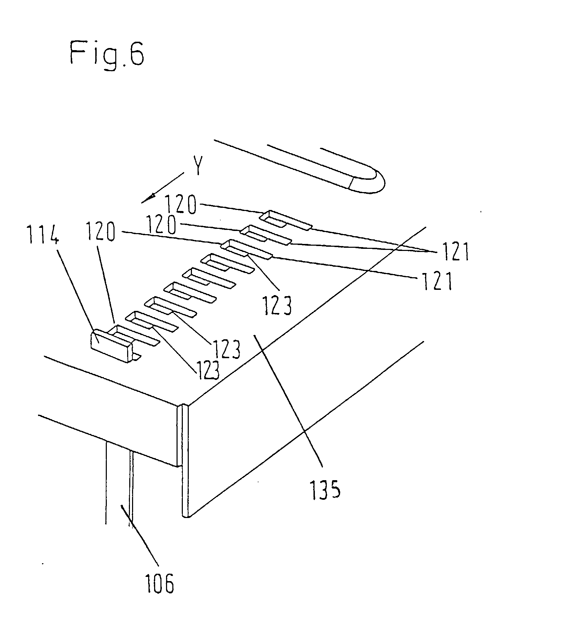

Die Rahmenteile 106 weisen der einen Außenkante zugeordnete, voneinander abgewandte hakenförmige Fortsätze 114 und 115 auf, mit deren Hilfe das Druckgehäuse auf einem Träger 135 über dort vorgesehene gestufte Durchbrüche 120 verriegelbar ist, wie dies insbesondere die Figuren 5 und 6 zeigen.

Die gestuften Durchbrüche 120 im Träger 135 sind am deutlichsten aus Figur 6 ersichtlich und umfassen jeweils einen längeren Teil 121 und einen kürzeren Teil 123, die in ihrer Breite je der Materialstärke eines Rahmenteiles entsprechen, wobei der erstere in seiner Länge der Länge der Fortsätze 114, 115 des Rahmenteils 106 und der letztere der Länge der Basis 132 der Fortsätze derart angepaßt sind, daß beim Einsetzen eines Rahmenteiles in die Durchbrüche im Bereiche des längeren Teiles und Verschieben der Rahmenteile in Richtung der y-Achse im Träger verriegelt sind, siehe Figur 6. In dieser Riegellage werden die Rahmenteile 106 durch Fortsätze 125 an der unteren Wandung 101 gehalten, die in entsprechende Durchbrüche 128 in dem Träger 135 in der in Figur 3 dargestellten Lage eingreifen.
Der Träger 135 dient, wie Figur 5 zeigt, als Chassis für die hier nicht näher dargestellte Sterilisationseinrichtung; vgl. Figur 1.

Die obere Wandung 102 trägt, wie Figur 3 zeigt, auf der dem Sterilisationsgefäß 130 zugewandten Seite eine zylinderförmige Metallfassung 146 zwecks Aufnahme einer mittels Federn 152 federnd in ihrer unwirksamen Lage gehaltenen Abdeckung 145, die aus einer eine Öffnung 151 für den Dampfzutritt aufweisenden Scheibe 138 besteht, die als Träger einer ihren Randbereich bildenden mit dem freien oberen Rand 134 des Sterilisationsgefäßes 130 korrespondierenden elastischen Gummidichtung 143 dient. Diese Gummidichtung ist wie Figur 3 zeigt, im Querschnitt etwa L-förmig ausgebildet, deren die Basis bildender Schenkel 148 die Scheibe 138 der Abdeckung umfaßt und eine Dichtfläche für das Sterilisationsgefäß bildet, während der andere Schenkel 149 eine an der seitlichen Innenfläche der Metallfassung 146 anliegende Führungsfläche bildet und eine Dichtungslippe 147 aufweist.

Zwecks dichtendem Aufeinandergleitens ist also die Metallfassung 146 zylinderförmig und die damit korrespondierende Abdeckung 145 kolbenförmig in jeweils etwa rechteckiger Form ausgebildet; vgl. Figur 2.

Da die Scheibe 138 zur überführung von Heißdampf eine öffnung 151 aufweist, ist die dem Sterilisationsgefäß 130 zugewandte und die der Oberwandung 101 zugewandte Fläche der Abdeckung 145 in unterschiedlicher Größe ausgebildet, um bei Dampfzufuhr durch den dann entstehenden unterschiedlichen Flächendruck das Sterilisationsgefäß 90 über die Abdeckung druckdicht geschlossen zu halten.

Die Abdeckung 145 bewegt sich also unter dem Einfluß des Dampfes nach unten, entgegen der Wirkung der Feder, und, sobald der Sterilisationsprozeß beendet ist und der Dampf entwichen ist, unter dem Einfluß der Feder nach oben, so daß das Sterilisationsgefäß 130 freigegeben wird.

Die Rahmenteile 106 sind aus Metall und auf einfache Weise sowohl bezüglich der dem nicht dargestellten Sterilisationsgerät zugeordneten Sterilisationskammer als auch bezüglich des Durchbruches 105 paßgenau in Serie herstellbar. Dies trifft in gleicher Weise für die aus Kunststoff bestehenden Platten 101 bis 104 zu.

Die beschriebene Ausbildung hat darüber hinaus im Rahmen der fertigungstechnischen Montage in einer Hilfsvorrichtung den Vorzug, daß die Platten 101 bis 104 in der Reihenfolge 101, 102 und 103, 104 in die Durchbrüche 105 zu den Innenkanten 161 bis 164 orientiert eingeführt und anschließend mit deren von den Vorsprüngen 107 gebildeten Nuten an die Innenkanten der Durchbrüche 105 der Rahmenteile 106 angedrückt werden, so daß die Nuten die Innenkanten der Durchbrüche 105 umgreifen. Nach Einsetzen des so gebildeten Druckgehäuses in die Durchbrüche 120 des Trägers 135 und Verschieben in y-Richtung sowie Eindrücken der Fortsätze 125 der Wandung 101 in die Durchbrüche 128 des Trägers 135 ist dieses mit dem Träger formschlüssig verbunden.

Auf diese verblüffend einfache Weise erlaubt die beschriebene Ausführung eine schnelle Montage, wobei einfach herzustellende, paßgenaue und standardisierte Bauteile Anwendung finden.

## Patentansprüche

1. Als Sterilisationskammer ausgebildetes Druckgehäuse (100) für die Aufnahme eines Sterilisationsgefäßes, **dadurch gekennzeichnet,** daß die Sterililsationskamer (100) Ober-, Unter- und Seitenwandungen aus einzelnen losen, wärmeisolierenden Platten (101, 102, 103, 104) aufweist, die in zentrischen Durchbrüchen (105) mindestens zweier identisch ausgebildeter, in einem Träger (135) formschlüssig gehaltener Rahmenteile (106) angeordnet und an den Innenkanten (161 bis 164) der Durchbrüche anliegend formschlüssig gehalten sind und daß die als Oberwandung dienende Platte (101) eine Metallfassung (146) trägt zwecks Aufnahme einer federnd in ihrer unwirksamen Lage gehaltenen Abdeckung (145) mit einer eine öffnung (151) für den Dampfübertritt aufweisenden Scheibe (138) als Träger einer ihren Randbereich bildenden, mit dem freien oberen Rand (134) des von der Sterilisationskammer (100) umfaßten Sterilisationsgefäßes (130) korrespondierenden elastischen Gummidichtung (143).

2. Druckgehäuse nach Anspruch 1, **dadurch gekennnzeichnet,** daß die Rahmenteile (106) der einen Außenkante zugeordnete voneinander abgewandte hakenförmige Fortsätze (114, 115) tragen, denen in ihren Längenausdehnungen gestufte Durchbrüche (120) des Trägers (103) derart zugeordnet sind, daß die Fortsätze nach dem Einsetzen in den jeweils längeren Teil (121) der Durchbrüche (120) und nach Verschieben quer zu den Durchbrüchen (y-Richtung) jeweils kürzeren Teil (123) der Durchbrüche (120) untergreifen und in dieser Lage durch Fortsätze (125) an der Unterwandung der Teil der Sterilisationskammer bildenden losen Platte (101), die in entsprechende Durchbrüche (128) in dem Träger (103) eingreifen, gehalten sind.

3. Druckgehäuse nach Anspruch 2, **dadurch gekennzeichnet,** daß die maximale Breite der Durchbrüche (120) der doppelten Materialstärke der Rahmenteile (106) und die Länge der längeren Teile (121) der Breite der hakenförmigen Fortsätze (110, 111) und die Länge der kürzeren Teile (123) der Breite der Basis (122) der Fortsätze (110, 111) entsprechen.

4. Druckgehäuse nach den Ansprüchen 1 bis 3, **dadurch gekennzeichnet,** daß die Platten (101 bis 104) die Innenkanten (161 bis 164) der Durchbrüche (105) der Rahmenteile (106) im montierten Zustand umgreifende von Vorsprüngen (107) gebildete Nuten aufweisen.

5. Druckgehäuse nach Anspruch 1, **dadurch gekennzeichnet,** daß die dem Sterilisationsgefäß (130) zugewandte und die der Metallfassung (146) zugewandte Fläche der Abdeckung (145) unterschiedliche Größe aufweisen.

6. Druckgehäuse nach Anspruch 1, **dadurch gekennzeichnet,** daß zwecks dichtenden Aufeinandergleitens die Metallfassung (146) zylinderförmig und die Abdeckung (145) kolbenförmig von jeweils etwa rechteckiger Form ausgebildet sind.

7. Druckgehäuse nach einem oder mehreren der Ansprüche 1 bis 6, **dadurch gekennzeichnet,** daß die Platten (101 bis 104) aus Kunststoff und die Rahmenteile (106) aus Metall bestehen.

## Claims

1. Pressure housing designed as a sterilisation chamber (100) for receiving a sterilisation vessel, characterised in that the sterilisation chamber (100) has upper, lower and side walls made from individual loose heat insulating plates (101, 102, 103, 104) which are arranged in central openings (105) of at least two identical frame parts (106) held with interlocking fit in a support (135) and are held so as to rest with interlocking fit on the inner edges (161 to 164) of the openings, and in that the plate (101) serving as an upper wall bears a metal mount (146) for the purpose of receiving a covering (145) held resiliently in its inactive position with a disc (138) having an opening (151) for the transfer of steam as a support for a corresponding elastic rubber seal (143) forming its edge region with the free upper edge (134) of the sterilisation vessel (130) enclosed by the sterilisation chamber (100).

2. Pressure housing according to claim 1, characterised in that the frame parts (106) of one external edge bear associated hook-shaped projections (114, 115) which are turned away from one another, with which openings (120) of the support (103) which are stepped in their longitudinal dimensions are associated in such a way that after being introduced into the longer part (121) of the openings (120) in each case and after being pushed transversely to the openings (y-direction), the projections engage in the shorter part (123) of the openings (120) and are held in this position by projections (125) on the lower wall of the loose plate (101) forming part of the sterilisation chamber, which projections engage in corresponding openings (128) in the support (103).

3. Pressure housing according to claim 2, characterised in that the maximum width of the openings (120) corresponds to twice the thickness of the frame parts (106) and the length of the longer parts (121) corresponds to the width of the hook-shaped projections (110, 111) and the length of the shorter parts (123) corresponds to the width of the base (122) of the projections (110, 111).

4. Pressure housing according to claims 1 to 3, characterised in that the plates (101 to 104) have grooves formed by projections (107) which in the assembled condition encompass the inner edges (161 to 164) of the openings (105) of the frame parts (106).

5. Pressure housing according to claim 1, characterised in that the face of the covering (145) facing the sterilisation vessel (130) and the face of the covering (145) facing the metal mount (146) have different sizes.

6. Pressure housing according to claim 1, characterised in that the metal mount (146) is cylinder-shaped and the covering (145) is cone-shaped with an approximately rectangular shape in each case so that they can slide on one another in a sealing manner.

7. Pressure housing according to one or more of claims 1 to 6, characterised in that the plates (101 to 104) consist of plastics material and the frame parts (106) consist of metal.

## Revendications

1. Boîte à pression formant chambre de stérilisation (100) destinée à recevoir un récipient de stérilisation, caractérisée par le fait que la chambre de stérilisation (100) présente des parois supérieure, inférieure et latérales constituées de plaques isolantes thermiques libres séparées (101, 102, 103, 104) qui sont placées dans des jours centraux (105) d'au moins deux parties de cadre identiques (106) tenues par emboîtement dans un support (135) et sont tenues par emboîtement appuyées contre les côtés intérieurs (161 à 164) des jours, et que la plaque (102) servant de paroi supérieure porte une monture métallique (146) destinée à recevoir une couverture (145) tenue élastiquement dans sa position inactive et comportant un disque (138), présentant une ouverture (151) pour le passage de vapeur, comme support d'un joint d'étanchéité élastique en caoutchouc (143) formant sa zone de bord et correspondant avec le bord supérieur libre (134) du récipient de stérilisation (130) enveloppé par la chambre de stérilisation (100).

2. Boîte à pression selon la revendication 1, caractérisée par le fait que les parties de cadre (106) portent des appendices en forme de crochet opposés (114, 115) associés à un côté extérieur et auxquels des trous étagés dans leur étendue longitudinale (120) du support (135) sont associés de façon telle que les appendices, après engagement dans la partie longue (121) des trous (120) et déplacement en travers des trous (direction y), soient appliqués sous la partie courte (123) des trous (120), et sont tenues dans cette position par des appendices (125) prévus sur la paroi inférieure de la plaque libre (101) faisant partie de la chambre de stérilisation qui s'engagent dans des trous correspondants (128) du support (135).

3. Boîte à pression selon la revendication 2, caractérisée par le fait que la largeur maximale des trous (120) correspond au double de l'épaisseur des parties de cadre (106), la longueur des parties longues (121) correspond à la largeur des appendices en forme de crochet (114, 115), et la longueur des parties courtes (123) correspond à la largeur de la base (132) des appendices (114, 115).

4. Boîte à pression selon les revendications 1 à 3, caractérisée par le fait que les plaques (101 à 104) présentent des rainures formées par des saillies (107) et embrassant à l'état monté les côtés intérieurs (161 à 164) des jours (105) des parties de cadre (106).

5. Boîte à pression selon la revendication 1, caractérisée par le fait que la surface de la couverture (145) dirigée vers le récipient de stérilisation (130) et la surface de celle-ci dirigée vers la monture métallique (146) sont de grandeur différente.

6. Boîte à pression selon la revendication 1, caractérisée par le fait que pour glisser l'une sur l'autre en assurant l'étanchéité, la monture métallique (146) est en forme de cylindre et la couverture (145) est en forme de piston, les deux étant de forme à peu près rectangulaire.

7. Boîte à pression selon une ou plusieurs des revendications 1 à 6, caractérisée par le fait que les plaques (101 à 104) sont en plastique et les parties de cadre (106) sont en métal.
